## Europäisches Patentamt

## European Patent Office

## Office européen des brevets

(19)

(11) Veröffentlichungsnummer: **0 067 408**
**B1**

(12) **EUROPÄISCHE PATENTSCHRIFT**

(45) Veröffentlichungstag der Patentschrift:
**27.03.85**

(21) Anmeldenummer: **82105031.7**

(22) Anmeldetag: **08.06.82**

(51) Int. Cl.⁴: **C 07 D 307/60** // C07C69/716

(54) Verfahren zur Herstellung von Tetronsäure.

(30) Priorität: **17.06.81 CH 3982/81**

(43) Veröffentlichungstag der Anmeldung:
**22.12.82 Patentblatt 82/51**

(45) Bekanntmachung des Hinweises auf die Patenterteilung:
**27.03.85 Patentblatt 85/13**

(84) Benannte Vertragsstaaten:
**AT BE CH DE FR GB IT LI NL SE**

(56) Entgegenhaltungen:
**EP - A - 0 018 162**
**DE - A - 2 413 709**

**TETRAHEDRON, Band 34, Nr. 2, 1978, Seiten 223-231, Oxford (GB); J.L. VAN DER BAAN et al.: "The total synthesis of the antibiotic malonomicin (K16)"**

(73) Patentinhaber: **LONZA AG, Gampel/Wallis (CH)**

(72) Erfinder: **Raimund, Miller, Dr., Berkshire Place 66, Hackensack, N.J. 07601 (US)**
Erfinder: **Leander, Tenud, Dr., Balfrinstrasse 23, VISP (Kanton Wallis) (CH)**

(74) Vertreter: **Barz, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

## Beschreibung

Die Erfindung betrifft ein Verfahren zur Herstellung von Tetronsäure aus 4-Halogenacetessigester.

Es ist bekannt, Tetronsäure, die unter anderem als Beschleuniger für die photographische Entwicklung verwendet wird, aus 4-Monohalogenacetessigester oder -säure herzustellen. Nach CH-PS 503 722 wird 4-Chloracetessigester mit einem aromatischen Amin zum 3-Arylaminocrotolacton umgesetzt und aus diesem durch Verteilung mit Mineralsäuren die Tetronsäure in Freiheit gesetzt. Der Nachteil dieser Methode besteht darin, daß die Isolierung der Tetronsäure nur durch Hochvakuumsublimation zu realisieren ist.

Nach CH-PS 529 128 wird von 4-Halogenacetessigsäure ausgegangen, die in wäßriger Lösung mit Alkalien umgesetzt wird. Durch Behandeln mit Mineralsäuren wird die Tetronsäure in Freiheit gesetzt. Auch hier muß die Isolierung der Tetronsäure über eine Hochvakuumsublimation erfolgen; außerdem ist die erzielbare Ausbeute nur 43 bis 44%.

Ziel der vorliegenden Erfindung ist es, diese Nachteile zu vermeiden.

Erfindungsgemäß wird dies dadurch erreicht, daß man den 4-Halogenacetessigester in den entsprechenden 4-tertiär-Butoxyacetessigester überführt und diesen durch cyclisierende Ätherspaltung in die Tetronsäure überführt.

Die cyclisierende Ätherspaltung kann einerseits durch Temperaturbehandlung des tertiär-Butoxyacetessigesters bei Temperaturen von über 100°C, vorzugsweise bei Temperaturen von 150 bis 280°C, andererseits durch Behandlung des tertiär-Butoxyacetessigesters mittels Säuren, wie Salzsäure, Trifluoressigsäure, saure Kationenaustauscher, vorzugsweise bei Temperaturen von 0 bis 30°C durchgeführt werden.

Wird die cyclisierende Ätherspaltung durch Temperaturbehandlung durchgeführt, kann das in einem Röhrenreaktor mit oder ohne Füllkörper oder in einem Dünnschichtreaktor geschehen. Der tertiär-Butoxyacetessigester kann sowohl flüssig als auch in Dampfform aufgegeben werden.

Als 4-Halogenacetessigester kommen die 4-Brom- und 4-Chlorderivate, vorzugsweise die 4-Chlorderivate, zum Einsatz. Als Ester werden zweckmäßig solche von Alkoholen mit 1 bis 6 C-Atomen, wie Methyl-, Äthyl-, Propyl-, Butylalkohol, verwendet. Vorzugsweise wird der 4-Chloracetessigsäureäthylester als Ausgangsprodukt verwendet.

Die Überführung des 4-Halogenacetessigesters in den tertiär-Butoxyacetessigester ist aus Tetrahedron, Bd. 34, Nr. 2, 1978, S. 223—31 bekannt und erfolgt vorzugsweise durch Einwirkenlassen eines Alkalisalzes, vorzugsweise des Natriumsalzes, des tertiär-Butylalkohols auf den 4-Halogenacetessigester in einem organischen Lösungsmittel. Als Lösungsmittel können alle geeigneten Verbindungen Verwendung finden, vorteilhaft aber werden Dimethylsulfoxid und Tetrahydrofuran verwendet. Die Reaktionstemperatur wird zweckmäßig bei 0 bis 50°C gehalten.

Eine bevorzugte Ausführungsform besteht darin, daß man von Natriumhydrid ausgeht, dieses in Tetrahydrofuran suspendiert und den tertiär-Butylalkohol zudosiert. In die so erhaltene Lösung wird der γ-Halogenacetessigester, gelöst in Tetrahydrofuran, zudosiert. Die dabei zweckmäßigste Reaktionstemperatur liegt bei 0 bis 50°C.

Nach beendeter Reaktion kann das Tetrahydrofuran durch Destillation zurückgewonnen werden. Der nach dieser Arbeitsweise hergestellte tertiär-Butoxyacetessigsäureäthylester ist ein viskoses gelbliches Öl mit einem $Kp_{0,3}$ von 61 bis 65°C.

### Beispiel 1

21,3 g 80%iges Natriumhydrid wurden durch dreimaliges Waschen mit je 30 ml Petroläther (Kp 40 bis 60°C) vom Weißöl befreit und zu 300 ml Tetrahydrofuran gegeben. Unter Rühren wurden nun 25,57 g tertiär-Butylalkohol so zudosiert, daß eine Reaktionstemperatur von 40°C eingehalten wurde. Nach beendeter Wasserstoffentwicklung wurde während 25 Minuten eine Lösung von 49,38 g (95,5%ig) 4-Chloracetessigäthylester in 120 ml Tetrahydrofuran zugetropft. Nach 20stündigem Rühren bei Raumtemperatur wurden 250 ml Tetrahydrofuran am Vakuumrotationsverdampfer abgedampft und der noch fließbare Rückstand in dünnem Strahl in eine Mischung von 45 g konz. HCl in 250 g Eiswasser gegossen, wobei sich der pH nach Ende der Zugabe auf 5 einstellte. Nun wude 4mal mit Äther extrahiert und nach Waschen und Trocknen der Äther am Vakuumrotationsverdampfer abgedampft. Der Rückstand wurde destilliert. Es resultierten 47,09 g (75,5%) 4-tertiär-Butoxyacetessigsäureäthylester.

5 g dieses Esters wurden in 10 ml 18%iger HCl gelöst und während 6 Stunden bei Raumtemperatur gerührt. Anschließend wurde die HCl am Vakuumrotationsverdampfer bei Temperaturen unter 30°C abgezogen, der kristalline Rückstand in wenig Wasser gelöst und das Wasser am Vakuumrotationsverdampfer bei Temperaturen unter 30°C wieder abgezogen. Diese Operation wurde noch einmal wiederholt. Es resultierten 2,48 g Tetronsäure in einer Reinheit von 99,1%, das entsprach einer Ausbeute von 100%, bezogen auf den 4-tertiär-Butoxyester. Die Gesamtausbeute, bezogen auf den 4-Chlorester, betrug 75,5%.

Der tertiär-Butoxyacetessigester hat folgende Charakteristik:

IR (dünner Film)
    Banden: 2990 (vs), 1750 (vs), 1730 (vs),
    1660 (m), 1370 (m), 1325 (m), 1105 (m) cm⁻¹.

NMR (10% in CCl₄)
$\delta$ = 1,2 (S)  }
$\delta$ = 1,29 (t) } 12 H
$\delta$ = 4,20 (q, 2H) ppm
$\delta$ = 3,45 (S, 2H)
$\delta$ = 3,93 (S, 2H)

### Beispiel 2

Wie in Beispiel 1 wurde der 4-Chloracetessigsäure-isopropylester mit Natrium-tertiär-butylat
zum 4-tertiär-Butoxyacetessigsäure-isopropylester umgesetzt, welcher wie in Beispiel 1 durch
Behandlung mit 18%iger Salzsäure in Tetronsäure überführt wurde. Ausbeute 77%.

### Beispiel 3

Wie in Beispiel 1 wurde der 4-Chloracetessigsäure-n-butylester mit Natrium-tertiär-butylat
zum 4-tertiär-Butoxyacetessigsäure-n-butylester
umgesetzt, welcher wie in Beispiel 1 durch Behandlung mit 18%iger Salzsäure in Tetronsäure
überführt wurde. Ausbeute 75,5%.

### Patentanspruch

Verfahren zur Herstellung von Tetronsäure aus
4-Halogenacetessigester durch Überführen des
4-Halogenacetessigesters in den entsprechenden 4-tertiär-Butoxyacetessigester, dadurch gekennzeichnet, daß man den 4-tertiär-Butoxyacetessigester durch cyclisierende Ätherspaltung,
die thermolytisch bei Temperaturen von über
100°C oder durch Behandeln mit Säure erfolgen
kann, zu Tetronsäure umsetzt.

### Claim

A process for the preparation of tetronic acid
from 4-halogeno acetoacetic acid ester by converting said 4-halogeno acetoacetic acid ester to
the corresponding 4-tertiary-butoxy acetoacetic
acid ester, characterized in that said 4-tertiary-
butoxy acetoacetic acid ester is converted to tetronic acid by cyclisating ether cleavage which
can be carried out thermolytically at temperatures above 100°C or by acid treatment.

### Revendication

Procédé pour la préparation d'acide tétronique à partir d'esters d'acides 4-halogénoacétyla-
cétiques par transformation de l'ester d'acide
4-halogénoacétylacétique en l'ester d'acide
4-tertio-butoxyacétylacétique correspondant,
caractérisé en ce qu'on transforme l'ester
d'acide 4-tertio-butoxyacétylacétique par clivage cyclisant d'éther qui peut se produire thermolytiquement à des températures supérieures
à 100°C ou par traitement avec des acides, en
acide tétronique.